# EUROPÄISCHE PATENTANMELDUNG

(11) **EP 3 088 032 A1**
(43) Veröffentlichungstag der Anmeldung: **02.11.2016**
(21) Anmeldenummer: 15001240.9
(22) Anmeldetag: 27.04.2015
(51) Int. Cl.: A61M 16/10, A61M 16/20, A61M 16/12, A61M 16/00

(54) **LACHGASMISCHER ZUR ERZEUGUNG EINES LACHGASGEMISCHES**

(71) Anmelder: Baldus Medizintechnik GmbH, 56182 Urbar (DE)
(72) Erfinder: Baldus, Fabian, 56179 Vallendar (DE)
(74) Vertreter: Hannke, Christian

(57) **Zusammenfassung**

Die Erfindung betrifft einen Lachgasmischer (100) zur Erzeugung eines Lachgasgemisches (13), umfassend:
- zumindest eine Mischkammer (1), innerhalb derer ein Sauerstoffgas (11) und ein Lachgas (12) in jeweils vorgebbaren Volumina und/oder Molanteilen miteinander zu dem Lachgasgemisch vermischt werden, wobei
- die Mischkammer zumindest einen Lachgasanschluss (120) und zumindest einen Sauerstoffgasanschluss (110) aufweist, so dass über entsprechend an diesen Anschlüssen montierte Gasleitungen (110A, 120A) sowohl das Sauerstoff- als auch das Lachgas in die Mischkammer eingeleitet wird,
- zumindest einen Konzentrationsregler (2), welcher dazu eingerichtet und dafür vorgesehen ist, ein Mischungsverhältnis zwischen dem in die Mischkammer eingeleiteten Sauerstoffgas und/oder dem in die Mischkammer eingeleiteten Lachgas vorgebbar einzustellen,
- zumindest einen Flussmengenregler (3), mittels dessen ein Volumenstrom der beiden Gase jeweils separat und/oder gemeinsam eingestellt werden kann,
wobei zur Notflutung der Mischkammer mit dem Sauerstoffgas und/oder mit Umgebungsluft an der Mischkammer zumindest ein O₂-Notfallknopf (4) angebracht ist, der in einer Betätigungsrichtung (41) eindrückbar ist, wobei eine, vorzugsweise ebene, Montagefläche (42) des O₂-Notfallknopfes, zu welcher die Betätigungsrichtung an zumindest einem Punkt in der Montagefläche senkrecht verläuft, frei von weiteren Regelelementen, ist.

## Beschreibung

Die vorliegende Erfindung betrifft einen Lachgasmischer zur Erzeugung eines Lachgasgemisches gemäß dem Oberbegriff des Patentanspruches 1.

Der hier vorgestellte Lachgasmischer umfasst zumindest eine Mischkammer, innerhalb derer ein Sauerstoffgas und ein Lachgas in jeweils vorgebbaren Volumina und/oder Molanteilen miteinander zu dem Lachgasgemisch vermischt werden.

Dabei umfasst die Mischkammer zumindest einen Lachgasanschluss und zumindest einen Sauerstoffgasanschluss, so dass über entsprechend an diesen Anschlüssen montierte Gasleitungen sowohl das Sauerstoff- als auch das Lachgas in die Mischkammer eingeleitet werden.

Zudem umfasst der Lachgasmischer zumindest einen Konzentrationsregler, welcher dazu eingerichtet und dafür vorgesehen ist, ein Mischungsverhältnis zwischen dem in die Mischkammer eingeleiteten Sauerstoffgas und/oder dem in die Mischkammer eingeleiteten Lachgas vorgebbar einzustellen.

Des Weiteren umfasst der hier beschriebene Lachgasmischer zumindest einen Flussmengenregler, mittels dessen ein Volumenstrom der beiden Gase jeweils separat und/oder gemeinsam eingestellt werden kann.

Derartige Lachgasmischer zur Erzeugung eines Lachgasgemisches sind jedoch bereits aus dem Stand der Technik bekannt.

Bekannt sind dabei insbesondere auch solche Lachgasmischer, welche einen O₂-Notfallknopf umfassen. Mittels eines solchen O₂-Notfallknopfes kann Patienten im Fall einer durch das Lachgas erzielten Übersedierung oder im Fall von Kreislaufproblemen reiner Sauerstoff, insbesondere in die Mischkammer, eingeleitet werden. Denkbar ist jedoch auch, dass durch Betätigung des O₂-Notfallknopfs der Stickstoffanteil auf einen vorgegebenen Volumenprozentsatz, beispielsweise auf 5 Volumen-%, relativ zum Sauerstoffvolumen reduziert wird.

Der O₂-Notfallknopf ist nämlich ein solcher, welcher die Zugabe von Stickstoff vorzugsweise vollständig unterbindet und somit einen Stickstoffeinfluss des Lachgases in die Mischkammer verhindert. Der Patient kann insofern innerhalb kürzester Zeit mit reinem Sauerstoff versorgt werden. Es versteht sich von selbst, dass anstatt reinem Stickstoff auch beliebige andere Stickstoffverbindungen, beispielsweise Distickstoffmonoxid, in Frage kommen.

Allerdings weisen aus dem Stand der Technik bekannte Lachgasmischer nur solche O₂-Notfallknöpfe auf, welche versteckt beispielsweise hinter einem Konzentrations- und/oder einem Flussmengenregler angeordnet sind. Im Falle einer Übersedierung ist daher die Erfahrung gemacht worden, dass - obwohl ein O₂-Notfallknopf vorhanden ist - dieser oft übersehen wurde und aus diesem Grunde wertvolle Zeit verstrichen ist, bis der Benutzer den O₂-Notfallknopf auffinden konnte.

Ausgehend hiervon ist es daher eine Aufgabe der vorliegenden Erfindung, das oben genannte Problem zu lösen und insofern einen Lachgasmischer zur Erzeugung eines Lachgasgemisches anzubieten, bei dem es in besonders einfacher Art und Weise möglich ist, im Falle einer Übersedierung eines Patienten oder im Falle von Kreislaufproblemen des Patienten eine Notfall-Sauerstoffflutung in der Mischkammer des Lachgasmischers vorzunehmen.

Diese Aufgabe wird durch den Gegenstand des Patentanspruches 1 gelöst.

Um nun diese Aufgabe zu lösen und die oben genannten Probleme zu vermeiden, macht die vorliegende Erfindung daher unter anderem von der Idee Gebrauch, dass zur Notflutung der Mischkammer mit dem Sauerstoffgas an der Mischkammer zumindest ein O₂-Notallknopf angebracht ist, der in einer Betätigungsrichtung eindrückbar ist, wobei eine, vorzugsweise ebene, Montagefläche des O₂-Notfallknopfes, zu welcher die Betätigungsrichtung an zumindest einem Punkt in der Montagefläche senkrecht verläuft, frei von weiteren Regelelementen ist. Die Montagefläche ist daher beispielsweise diejenige Außenfläche eines Mischkammergehäuses, an welcher der O₂-Notfallknopf angeordnet ist. Die Montageflache kann jedoch auch eine diejenige Außenfläche einer Steuerungseinheit des Lachgasmischers sein, wobei eine solche Steuerungseinheit dann entfernt von dem eigentlichen Mischkammergehäuse angeordnet sein kann.

Dabei handelt es sich insbesondere bei einer ebenen Montagefläche um eine solche Montagefläche, welche frei von Spalten und Unterbrechungen ist. Insbesondere kann es sich jedoch bei einer ebenen Montagefläche daher um eine solche Fläche handeln, welche entweder krümmungsfrei ist oder aber zumindest eine Krümmung aufweist. Handelt es sich bei der Montagefläche um eine krümmungsfreie Fläche, stellt diese Montagefläche eine Montageebene dar. Auf einer solchen Montageebene verläuft daher die Betätigungsrichtung an jedem Punkt senkrecht.

Mit dem Begriff "Regeielemente" werden solche Elemente eines Lachgasmischers bezeichnet, welche zur Einstellung eines Mischungsverhältnisses und/oder zur Einstellung einer Flussmenge der Gase geeignet sind. Ein Ein- und Ausschaltknopf des Lachgasmischers ist daher verschieden von einem solchen Regelelement. Mit anderen Worten ist denkbar, dass neben dem O₂-Notfallknopf in der Montagefläche zusätzlich noch ein solcher Ein- und Ausschaltknopf angeordnet ist.

Insofern ist daher in besonders einfacher Art und Weise gewährleistet, dass der hier beschriebene O₂-Notfallknopf nicht durch andere Regelelemente für den Benutzer verdeckt ist oder optisch in den Hintergrund gerät, sondern insbesondere in einem Zentralbereich der Montagefläche angeordnet ist.

Gemäß zumindest einer Ausführungsform umfasst der Lachgasmischer zur Erzeugung eines Lachgasgemisches zumindest eine Mischkammer, innerhalb derer ein Sauerstoffgas und ein Lachgas in jeweils vorgebbaren Volumina und/oder Molanteilen miteinander zu dem Lachgasgemisch vermischt werden, wobei die Mischkammer zumindest einen Lachgasanschluss und zumindest einen Sauerstoffgasanschluss aufweist, so dass über entsprechend an diesen Anschlüssen montierte Gasleitungen sowohl das Sauerstoff- als auch das Lachgas in die Mischkammer eingeleitet wird.

Zudem umfasst der hier beschriebene Lachgasmischer zumindest einen Konzentrationsregler, welcher dazu eingerichtet und dafür vorgesehen ist, ein Mischungsverhältnis zwischen dem in die Mischkammer eingeleiteten Sauerstoffgas und/oder dem in die Mischkammer eingeleiteten Lachgas vorgebbar einzustellen, wobei zumindest ein Flussmengenregler, mittels dessen ein Volumenstrom der beiden Gase jeweils separat und/oder gemeinsam eingestellt werden kann, ebenso ein Teil des hier beschriebenen Lachgasmischers ist.

Erfindungsgemäß wird zur Notflutung der Mischkammer mit dem Sauerstoffgas und/oder mit Umgebungsluft an der Mischkammer zumindest ein O₂-Notfallknopf angebracht, der in einer Betätigungsrichtung eindrückbar ist, wobei eine, vorzugsweise ebene, Montagefläche des O₂-Notfallknopfes, zu welcher die Betätigungsrichtung an zumindest einem Punkt in der Montagefläche senkrecht verläuft, frei von Regelelementen ist.

Mit anderen Worten handelt es hierbei erstmals um einen solchen (insbesondere analoge) Lachgasmischer, bei welchem ein Alarm einer Alarmeinrichtung dem Benutzer mitteilt, wenn der Patient mit zu wenig Sauerstoff versorgt wird und infolgedessen der Benutzer oder aber auch vollautomatisch der O₂-Notfallknopf betätigt wird um eine Zufuhr von Lachgas zu dem Patienten vollständig zu unterbinden, sodass dieser, insbesondere nur, mit Sauerstoff versorgt wird. Zum besonders schnellen Auffinden kann der Notfallknopf mit Rot gekennzeichnet sein.

Insbesondere kann in diesem Zusammenhang somit vorstellbar sein, dass wenn der 02 Alarm ertönt, dies bedeutet, dass der Mischer mit zu wenig 02 versorgt wird. In diesem Falle fließt auch vorzugsweise kein Lachgas mehr (100% Lachgas können sogar tödlich sein, Lachgassperre bei 02-Ausfall) und die leere 02-Flasche muss getauscht werden oder eine Steckverbindung zu einem Gas-(O2)-Versorgungsnetz hat sich getrennt oder das Versorgungsnetz ist leer.

Es ist somit vorstellbar, dass wenn der Alarm ertönt der 02-Flush-Knopf nicht mehr gedrückt werden kann oder nicht mehr gedrückt zu werden braucht.

Grundsätzlich kann jedoch der hier beschriebene Lachgasmischer auch zur vorgebbaren Mischung anderer Gase zur Anwendung kommen. Beispielsweise kann dann das Lachgas durch ein anderes Gas ersetzt sein um dann mit dem Sauerstoff vermischt zu werden. Es ist allerdings ebenso möglich den Sauerstoff selbst durch ein anderes Gas zu ersetzen.

Zudem ist vorstellbar, dass der hier beschriebene Lachgasmischer nicht nur im Rahmen einer Zahnbehandlung sondern alternativ oder zusätzlich auch im Rahmen eines sonstigen Sedierungsprozesses, beispielsweise vor oder während einer Geburtsphase im Kreissaal, Anwendung findet. In diesem Kontext ist vorstellbar, dass im Rahmen einer gynäkologischen Behandlung der Lachgasmischer über ein Demandventil mit einer Mund-Nasenmaske verfügt.

Insbesondere sollte jedoch herausgestellt werden, dass der hier beschriebene Lachgasmischer über zumindest einen Eingangsdruckminderer verfügen kann, mittels dessen ein konstanter Systemdruck (einzelne Gasdrücke oder Gesamtdruck in der Mischkammer) gewährleistet werden kann.

Gemäß zumindest einer Ausführungsform umfasst die Mischkammer eine Vorkammer sowie eine Hauptkammer, wobei nur die Vorkammer die beiden Gasanschlüsse umfasst und die Hauptkammer über zumindest eine Fluidverbindung mit der Vorkammer verbunden ist, wobei ein Raumluftventil anstatt an einer Halterung für die Hauptkammer befestigt zu sein abseits dieser Halterung angeordnet ist.

Aus dem Stand der Technik sind nämlich nur solche Raumluftventile bekannt, welche an oder oberhalb der Hauptkammer, insbesondere an einer Halterung für die Hauptkammer befestigt sind. Handelt es sich bei der Hauptkammer um einen Gasbeutel, so ist oftmals im Stand der Technik das Raumluftventil an dieser oberhalb der Hauptkammer angeordneten Halterung angeordnet.

Dies führte jedoch oftmals zu einem ungewollten Einführen von durch das Raumluftventil angesaugtem Blut, Speichel, etc.... Die über ein solches, dann über dem Gasbeutel angeordnetes Raumluftventil angesaugten Verunreinigungen kontaminieren oftmals Innenflächen der Hauptkammer. Es wurde daher die Erfahrung gemacht, dass dies zwar eine platzsparende Anordnung des Raumluftventils ist, jedoch über das Raumluftventil beim Ansaugen der Luft über das Raumluftventil Staub, Blut, Speichel, Reinigungsmittel, etc. somit in die Hauptkammer laufen können.

Insofern wurde nunmehr erdacht, dass dieses Raumluftventil abseits einer derartigen Hauptkammer und insbesondere abseits einer derartigen Halterung für die Hauptkammer plaziert wird. Beispielsweise ist ein derartiges Raumluftventil an einer von der Halterung für die Hauptkammer verschiedenen Halterung angeordnet. Insbesondere kann eine Flächennormale der Ventilöffnung des Raumluftventils nach unten und im Wesentlichen parallel zur Hauptersteckungsrichtung der Hauptkammer verlaufen. Bei dem Raumluftventil kann es sich um ein Unterdruckventil handeln, welches sich automatisch beim Einatmen des Patienten dann öffnet, wenn die Hauptkammer von dem Patienten leer geatmet ist.

Gemäß zumindest einer Ausführungsform sind zumindest der O₂-Notfallknopf, vorzugsweise jedoch auch der Konzentrationsregler und der Flussmengenregler, an der Vorkammer angeordnet. Insofern ist das System der Mischkammer unterteilt in ein Vorkammer- und ein Hauptkammersystem. Die Vorkammer ist dazu da, um ein in die Hauptkammer eingeleitetes Lachgas in seiner Zusammensetzung in Bezug auf den Sauerstoff- und den Lachgasanteil einzustellen, während aus der Hauptkammer das dann fertig zusammengestellte Lachgas in einen Entnahmeschlauch eingeleitet wird. Der Entnahmeschlauch kann dann mit einer entsprechenden Nasenmaske verbunden sein.

Gemäß zumindest einer Ausführungsform ist die Hauptkammer mit zumindest einem Gasbeutel gebildet. Insofern ist die Hauptkammer beispielsweise anstatt mit einem nicht deformierbaren Bauteil mit einem solchen Bauteil gebildet, welches zum einen nicht nur deformierbar ist, sondern sich darüber hinaus auch individuell in seiner Größe an die entsprechend benötigte Menge an Lachgas anpasst.

Gemäß zumindest einer Ausführungsform umfasst der hier beschriebene Lachgasmischer zumindest eine Mischersteuerungseinheit, wobei die Mischersteuerungseinheit zumindest eine digitale Steuerungseinheit umfasst, wobei die Steuerungseinheit derart ausgestaltet ist, dass diese den Konzentrationsregler und/oder den Flussmengenregler mittels der Vorgabe eines Benutzers steuert. Insbesondere kann die Mischersteuerungseinheit den Konzentrationsregler und/oder den Flussmengenregler auch regeln.

Mit anderen Worten sind also die hier beschriebene Mischersteuerungseinheit und insbesondere der hier beschriebene Lachgasmischer im Sinne der Anmeldung rein elektronisch gesteuert. Werden beispielsweise die digitale Steuerungseinheit und insbesondere ein Touchscreen dieser digitalen Steuerungseinheit durch den Benutzer bedient, steuert also rein elektronisch diese Steuerungseinheit den Konzentrationsregler und/oder den Flussmengenregler mit zumindest einer Stelleinheit an.

Alternativ hierzu kann es sich bei der Mischersteuerungseinheit auch um eine solche handeln, welche eine analoge Steuerungseinheit umfasst. Eine derartige analoge Steuerungseinheit ist daher frei von einem Touchscreen und wird durch den Benutzer beispielsweise händisch, insbesondere nur händisch, gesteuert. Diese Mischersteuerungseinheit umfasst daher die beiden Regler selbst. Dazu umfassen der Konzentrationsregler und/oder der Flussmengenregler Eingriffselemente, mit denen der Benutzer beispielsweise händisch die Konzentration und die Flussmenge der einzelnen Gase an den Reglern steuern kann.

Insbesondere kann jedoch ein solcher analoger Lachgasmischer umfassend die analoge Steuerungseinheit ein akustisches Warnmittel umfassen. Ein solches Warnmittel kann jedoch auch in dem obig beschriebenen digitalen Lachgasmischer angeordnet sein.

Sollte nämlich der Fluss eines oder beider Gase unterbrochen werden, ertönt unmittelbar nach der Unterbrechung des jeweiligen Gasflusses ein insbesondere akustisches Warnsignal.

Insofern verfügt der hier beschriebene Lachgasmischer über ein derartiges akustisches Alarmpaket. Geht beispielsweise die Sauerstoffflasche leer, so kann ein Alarm ertönen. Wenn nämlich die Sauerstoffzufuhr unterbrochen ist, fließt somit auch kein Stickstoff mehr in die Mischkammer.

Insbesondere kann neben einer Stickstoffzufuhrunterbrechung auch eine Lachgaszufuhrunterbrechung realisiert sein.

Gemäß zumindest einer Ausführungsform umfasst die digitale Steuerungseinheit zumindest einen Betätigungsbildschirm, insbesondere einen Touchscreen, mittels dessen über eine händische Betätigung des Benutzers elektronisch, vorzugsweise nur elektronisch, der Konzentrationsregler und/oder der Flussmengenregler betätigbar sind.

Einer der Vorteile der Realisierung der Steuerungseinheit mittels des Betätigungsbildschirmes ist, dass der Benutzer sich nur auf die Anzeige des Betätigungsbildschirmes konzentrieren muss, um den Lachgasmischer in seiner Gesamtheit steuern zu können. Dabei kann der Betätigungsbildschirm zeitaktuelle Anzeigen der Lachgas- und/oder Sauerstoffgaskonzentration innerhalb des Lachgasgemisches sowie einzelne Flussmengen in Litern pro Minute oder auch eine Gesamtflussmenge des Gasgemisches umfassen. Derartige Zahlen- und Mengenangaben können daher durch Betätigung des Betätigungsbildschirmes besonders individuell, also über den Betätigungsbildschirm selbst, eingestellt werden.

Gemäß zumindest einer Ausführungsform umfasst der Betätigungsbildschirm zumindest ein Einstellfeld, auf dem der Benutzer händisch eine Konzentrationsänderung des Sauerstoffgases und/oder des Lachgases in vorgebbaren Änderungsschritten einstellen kann, wobei die Konzentrationsänderung vorzugsweise nur durch die Betätigung des Einstellfeldes einstellbar ist. Dies bietet den Vorteil, dass mittels beispielsweise eines einmaligen Drückens auf das Einstellfeld (= Drückknopf), d.h. mittels des Drückens eines einzigen Knopfes, eine Konzentrationsänderung beispielsweise des Sauerstoffgases aber auch alternativ oder zusätzlich des Lachgases eingestellt werden kann. Standardmäßig kann daher der Lachgasmischer so eingestellt sein, dass der Betätigungsbildschirm darauf eingestellt ist, den Sauerstoff zu einer vorgegebenen Menge von Lachgas in 5 Prozentschritten hinzuzufügen.

Drückt nun der Benutzer auf dieses Einstellfeld (welches beispielsweise mit "Konzentrationsänderung: 1%/5%" benannt ist), so wird das Lachgas in ein Prozentschritten erhöht bzw. reduziert. Eine Umstellung ist daher jederzeit möglich, z.B. wenn man ein Kind sedieren möchte und das Lachgas fein titrieren muss.

Daneben ist jedoch auch denkbar, dass eine Patienten-ID in der digitalen Steuerungseinheit hinterlegt ist oder abgespeichert wird. Beispielsweise öffnet sich vor der Sedierung ein Tippfeld des Betätigungsbildschirmes mit Buchstaben und Ziffern, um einen Patienten genau mit Namen oder Patientennummer anlegen zu können, was entscheidende Vorteile für die Dokumentation der behandelnden Person mit sich bringt.

Zudem kann durch die Steuerungseinheit auf dem Behandlungsbildschirm ein Menüpunkt "letzte Patienten aufrufen" angezeigt sein. Dies kann von dem Benutzer auch angewählt werden, so dass nach dem Anwählen auf dem Betätigungsbildschirm die zuletzt behandelten Patienten angezeigt werden. Dies kann entweder mittels eines anonymisierten Kodierungssystems geschehen oder auch mit dem konkreten Namen bzw. der entsprechenden Patienten-ID.

Es können auch die Behandlungszeit, der Beginn der Sedierung, Datum und maximaler Lachgaswert angezeigt werden. Außerdem besteht die Möglichkeit, den Patienteneintrag zu löschen, wobei auf dem Betätigungsbildschirm angeordnete und erscheinende Pfeile zum Weiterklicken auf den nächsten Patienten dienen können.

Zudem können über den hier beschriebenen Betätigungsbildschirm neben einer Konzentrationsänderung noch weitere Einstellungen vorgenommen werden.

Diese weiteren Einstellungen können Uhrzeit/Datum umfassen. Dabei können nämlich Uhrzeit und Datum beliebig angepasst werden. Auch können eine Lautstärke des Alarms im Falle einer Zufuhrunterbrechung eines der Gase und entsprechende Tastentöne besonders einfach eingestellt und reguliert werden. Auch können entsprechende Tastentöne vollständig ausgestellt werden.

Zudem bietet der hier beschriebene Betätigungsbildschirm die Möglichkeit, eine Servicekultur für den Lachgasmischer zu etablieren. Möglich ist es nämlich, den letzten und den nächsten Servicetermin angezeigt zu bekommen. Außerdem ist es möglich, die Hardware/Software anhand ihrer Seriennummer des Gerätes zu identifizieren, was wiederum auf dem Betätigungsbildschirm angezeigt werden kann.

Standardmäßig kann daher auch der Lachgasmischer so ausgeliefert werden, dass auf dem Betätigungsbildschirm prozentual angezeigt wird, wie viel Volumenprozent Lachgas in die Hauptkammer, fließen. Drückt nun der Anwender auf "Anzeige O₂/N₂O", so wird die prozentuale Sauerstoffkonzentration auf dem Sedierungsdisplay angezeigt. Eine Umstellung ist jederzeit möglich.

Zudem kann die hier beschriebene digitale Steuerungseinheit einen Fehlerspeicher, der eine vorgegebene Anzahl von Fehlermeldungen, beispielsweise von 20 Fehlermeldungen des Gerätes anzeigt und speichert, vorhanden sein.

Es besteht zudem die Möglichkeit, den Speicher zu löschen. Bei der Wartung kann insbesondere über eine Master-SD-Karte/Dongl für Serviceeinsätze eine vorgegebene Anzahl von Fehlern, vorzugsweise alle Fehler, aufgeführt werden. Die Fehler sind beispielsweise in einem Administratormenü immer nachzulesen und zu exportieren. Insbesondere kann zudem der Behandler mit einem USB Stick und/oder einem SB-Port Protokolle z.B. mit einer Patientennummer speichern. Zudem kann der Behandler die Behandlungsdauer, die Konzentration etc. dokumentieren.

Zudem ist es möglich, dass die hier beschriebene digitale Steuerungseinheit internetfähig ist. Beispielsweise weist die hier beschriebene digitale Steuerungseinheit ein LAN- oder WLAN-Modul auf, mit dem sich die digitale Steuerungseinheit gemäß zumindest einem standardisierten Internetprotokoll mit einer anderen digitalen Steuerungseinheit und/oder mit einem entfernt platzierten Administrator verknüpfen kann. Beispielsweise kann ein derartiger Administrator daher über die LAN- oder WLAN-Verbindung sich in die digitale Steuerungseinheit von extern einloggen, um somit einen Remote-Support gewährleisten zu können. Auch ist es möglich, dass mittels einer derartigen Steuereinheit ein Administrator einen Boot oder Reboot oder eine Ferndiagnose oder auch Ferneinstellungen an der digitalen Steuerungseinheit vornehmen kann. Des Weiteren kann ein Fernbedienungsanschluss für eine Remotecontrol vorgesehen sein. Somit kann die digitale Steuerungseinheit einen Kabel- und/oder IR-und/oder Bluetoothanschluss umfassen und/oder über Funk betrieben sein.

Zudem kann der hier beschriebene Lachgasmischer neben dem O₂-Notfallknopf eine Lachgassperre, ein Limitierungselement der Lachgaskonzentration und unterschiedliche Anschlussgrößen über Lachgas und Sauerstoff aufweisen.

Weiter ist vorstellbar, dass an der Mischkammer zumindest ein Drucksensor angeordnet ist, welcher zumindest einen der Gasdrücke (Lachgas, Lachgasgemisch und/oder Sauerstoffgas) messen und über eine mit dem Drucksensor innerhalb eines Regelkreises verbauten Regeleinheit die einzelnen Gasdrücke während der Behandlung oder auch vorher einzustellen.

Es wäre auch möglich durch die Kenntnis des Flaschendrucks Rückschlüsse auf die mögliche Restlaufzeit durch den Rückschluss auf das zur Zeit der Behandlung verabreichte Gas/ Minute zu berechnen. (Meldung: z.B. "Bei gleichbleibendem Atemminutenvolumen/Flow noch ca. X Minuten zur Verfügung" oder "Achtung in weniger als 30 Minuten ist das Gas leer").

Des Weiteren kann neben dem Drucksensor insbesondere auch durch Einbeziehung zumindest eines der Flowmeter in den Regelkreis über einen Recheneinheit voraus berechnet werden wie lange die Abgabe eines entsprechenden Gases aus der jeweiligen Gasflasche noch unter eine vorgebbaren Druck aufrechterhalten werden kann.

Gemäß zumindest einer Ausführungsform ist der hier beschriebene Lachgasmischer frei von einem An- und Ausschaltknopf. Insofern kann vermieden werden, dass der Benutzer sich beispielsweise im Falle eines Notfalls nicht im Klaren ist, dass der Lachgasmischer über einen derartigen Ein- und Ausschaltknopf auszuschalten ist. Der hier beschriebene Lachgasmischer kann daher frei von einem derartigen An- und Ausschaltknopf und kann daher im Betrieb einfach durch Ein- und Ausstecken zu einer Steckdose an das Stromnetz angeschlossen oder von diesem getrennt werden.

Alternativ jedoch kann der Lachgasmischer trotzdem einen An- und Ausschaltknopf dann umfassen, wenn dieser An- und Ausschaltknopf derart ausgestaltet ist, dass dieser nur durch beständiges Drücken über einen vorgegebenen Zeitraum, beispielsweise zumindest zwei Sekunden, gedrückt bleibt, damit der Behandler nicht aus Versehen das Gerät ausschaltet, wenn er den 02-Notfallknopf betätigen will. Vorteilhaft befindet sich der Ein/Aus-Schalter nicht auf dem Touchscreen.
Insbesondere kann mittels des hier beschriebenen Lachgasmischers zudem ein Gesamtflow erhöht werden. Beispielsweise ermöglicht nämlich der hier beschriebene Lachgasmischer einen Gesamtfluss von 20l/min. oder höher, so dass die Hauptkammer, also beispielsweise der Atembeutel, stets ausreichend mit dem Lachgas gefüllt ist und die Sedierung wirken kann.

Ist nämlich ein derartiger Gesamtflow zu gering gewählt, beispielsweise im Bereich von nur 8 - 10 l/min., atmet häufig der Patient die Hauptkammer leer, weil ein aufgeregter Patient schneller und mehr l/min atmet und es öffnet sich somit das Raumventil, so dass die Umgebungsluft angesaugt wird und die Sedierung somit nicht wirken kann. Insbesondere hat sich nämlich ergeben, dass ein Volumen der Hauptkammer im Bereich von <101 auch deshalb oftmals zu gering gewählt ist, weil im Falle von aufgeregten Patienten ein eine sehr schnelle Atemfrequenz vorliegt

Wie bereits obig erwähnt, ist zudem eine Ablesbarkeit der Lachgaskonzentration im Vergleich zu dem Stand der Technik entscheidend verbessert. Im Vergleich zu bereits bekannten Lachgasmischern wird nämlich hier unter anderem auch vorgeschlagen, dass eine prozentuelle Lachgaskonzentration an der von dem Patienten in seiner Gesamtheit eingeatmeten Gasmenge angezeigt wird anstatt dass lediglich eine prozentuelle Sauerstoffgaskonzentration angezeigt wird. Somit muss die behandelnde Person nicht mehr umrechnen und es werden auch Fehleinstellungen vermieden.

Aus Sicherheitsgründen beginnt daher die hier vorgeschlagene Konzentrationsskala an Lachgas bei 0% Lachgas und endet bei 50% bzw. 60% bzw. 70% Lachgas (je nach Variante). Auch kann ein Gesamtflowanschlag und/oder ein separater Lachgaskonzentrationsregleranschlag an dem Lachgasmischer angeordnet sein, welcher Risiken bei der Bedienung des Gasflusses minimiert. Mittels eines derartigen Anschlages ist es nämlich vermieden, dass zu viel Gas fließt. Insbesondere kann der Gesamtflowanschlag es verhindern, dass mehr als z. B. 20 Liter pro Minute gemischtes Gas fließen.

Im Folgenden wird der hier beschriebene Lachgasmischer anhand von Ausführungsbeispielen und den dazugehörigen Figuren näher beschrieben:
Die Figuren 1A, 1B, 1C zeigen in einer schematischen Seitenansicht ein Ausführungsbeispiel eines hier beschriebenen analogen Lachgasmischers.
Die Figuren 2a und 2b zeigen in schematisch perspektivischen Ansichten ein Ausführungsbeispiel eines hier beschriebenen digitalen Lachgasmischers.

In den Figuren sind gleiche oder gleichwirkende Bestandteile jeweils mit den gleichen Bezugszeichen versehen. Die hier dargestellten Elemente sind nicht als maßstabsgerecht anzusehen, vielmehr können einzelne Elemente zum besseren Verständnis übertrieben groß dargestellt sein.

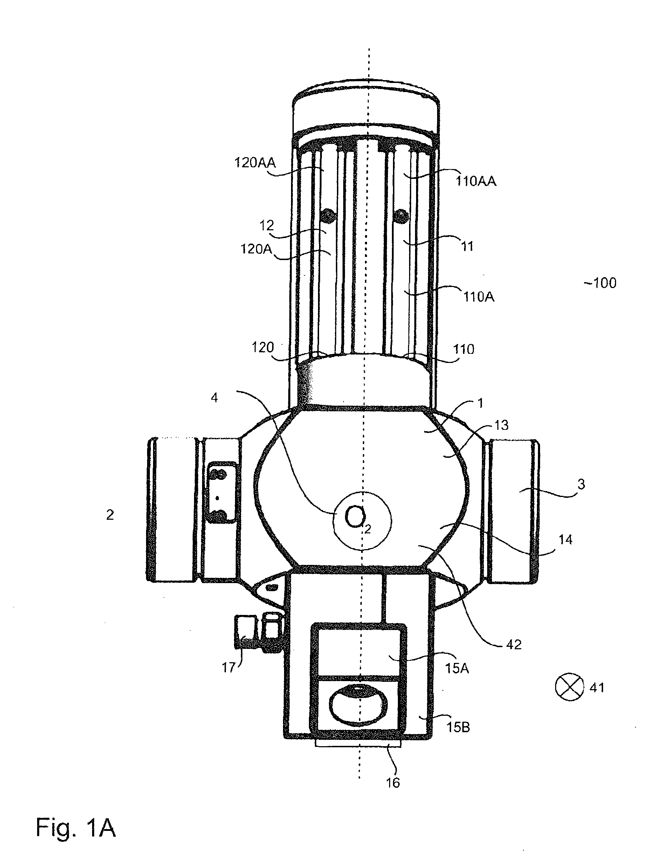

In der Figur 1A ist ein Ausführungsbeispiel eines hier beschriebenen analogen Lachgasmischers 100 gezeigt.

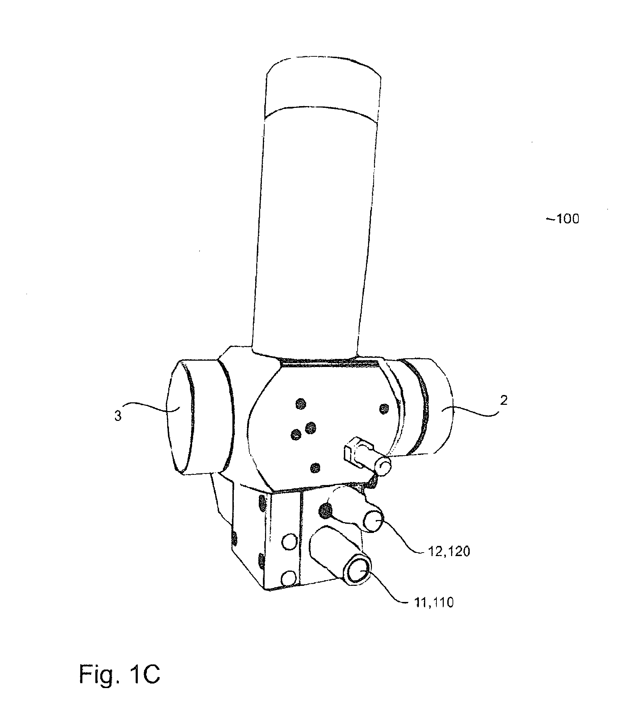

Aus dieser Figur ist besonders einfach erkennbar, dass der hier beschriebene Lachgasmischer 100 zur Erzeugung eines Lachgasgemisches zumindest eine Mischkammer 1 umfasst, innerhalb derer ein Sauerstoffgas 11 und ein Lachgas 12 in jeweils vorgebbaren Volumina und/oder Molanteilen miteinander zu dem Lachgasgemisch 13 vermischt werden, wobei die Mischkammer 1 einen Lachgasanschluss 120 (siehe Figur 1C) und einen Sauerstoffgasanschluss 110 (siehe Figur 1C) aufweist, so dass über entsprechend an diesen Anschlüssen montierte Gasleitungen 110A, 120A sowohl das Sauerstoff- als auch das Lachgas in die Mischkammer 1 eingeleitet werden. Insofern zeigt die Figur 1C eine perspektivische Rückansicht der in der Figur 1A gezeigten Vorderansicht des Lachgasmischers.

Insbesondere sind die Gasleitungen 110A (Sauerstoff), 120A (Stickstoff) derart ausgestaltet, dass diese jeweilige Flowmeter 110AA und 120AA umfassen, anhand derer die jeweilige Flussgeschwindigkeit der Gase über eine druckverschiebliche Flowkugel anhand der jeweiligen Messskalen abgelesen werden kann.

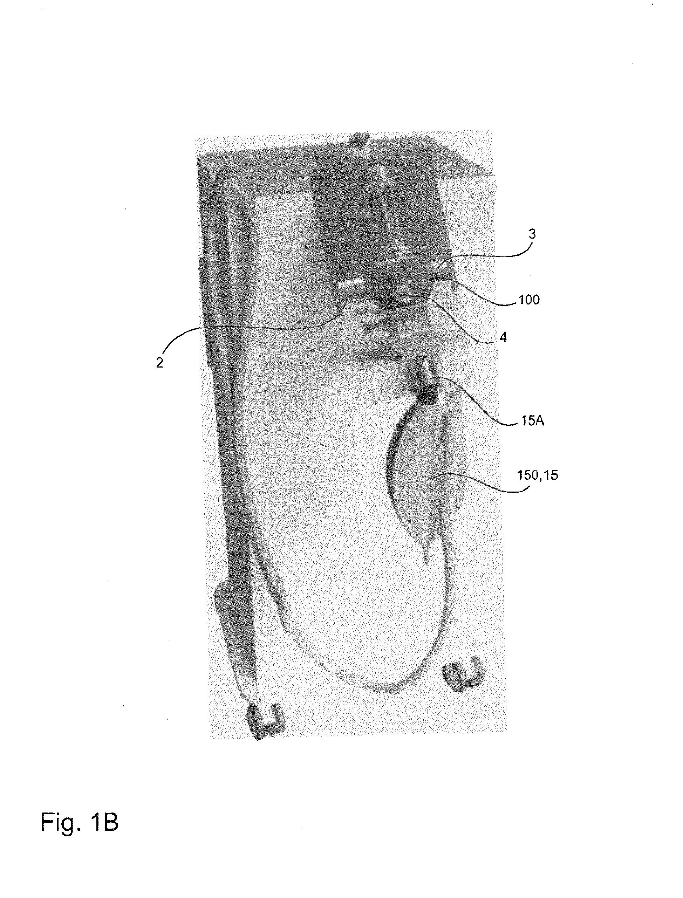

Zudem ist aus der Figur 1 ein Konzentrationsregler 2 erkennbar, mittels dessen ein Mischungsverhältnis zwischen dem in die Mischkammer 1 eingeleiteten Sauerstoffgas 11 und/oder dem in die Mischkammer 1 eingeleiteten Lachgas 12 vorgebbar eingestellt werden kann, wobei ebenso ein Flussmengenregler 3 erkennbar ist, mittels dessen ein Volumenstrom der beiden Gase 11, 12 separat und/oder gemeinsam eingestellt werden kann.

Besonders prominent aus der Figur 1 erkennbar ist der stets vorhandene und erfindungsgemäße O₂-Notfallknopf 4, welcher zur Notflutung der Mischkammer 1 mit dem Sauerstoffgas 11 und/oder mit Umgebungsluft dient.

Wird nun der O₂-Notfallknopf 4 gedrückt, gelangt in die Mischkammer 1 ausschließlich nur noch das Sauerstoffgas 11. Eine Zufuhr des Lachgases 12 ist daher vollständig verhindert. Dazu ist der O₂-Notfallknopf 4 an einem Mischkammergehäuse 1A der Mischkammer 1 angeordnet und in einer Betätigungsrichtung 41 eindrückbar, wobei das Mischkammergehäuse der Mischkammer 1 eine Montageebene 42 umfasst.

Der O₂-Notfallknopf 4 ist daher an dieser Montagefläche 42 mit der Mischkammer 1 wirkverbunden und auch in Richtung der Montagefläche 42 in der Betätigungsrichtung 41 eindrückbar.

Entscheidend ist nun, festzustellen, dass die Montagefläche 42 frei von weiteren Regelelementen ist. Dies heißt insbesondere, dass der Konzentrationsregler 2 und der Flussmengenregler 3 nicht an der Montagefläche 42 des Mischkammergehäuses der Mischkammer 1 angeordnet sind, sondern an davon verschiedenen Seiten des Mischkammergehäuses 1 A der Mischkammer 1 angeordnet sind. Dies hat unmittelbar zur Folge, dass die Anordnungsseite des O₂-Notfallknopfes 4 und die Anordnungsseiten des Konzentrationslegers 2 und/oder des Flussmengenreglers 3 an dem Mischkammergehäuse 1A der Mischkammer 1 voneinander verschieden sind. Insofern ist garantiert, dass in einem Notfall der Benutzer den O₂-Notfallknopf 4 sofort finden kann und dieser weder durch den Konzentrationsregler 2 noch durch den Flussmengenregler 3 oder weitere davon verschiedene Regelelemente verdeckt ist.

Aus der Figur 1B ist der in der Figur 1A beschriebene Lachgasmischer 100 in einer perspektivischen Ansicht erkennbar, wobei im Unterschied zu der Figur 1A nun besser erkennbar ist, dass die Mischkammer 1 mit einer Vorkammer 14 sowie mit einer Hauptkammer 15 gebildet ist. Die Hauptkammer 15 bildet einen Gasbeutel 150 aus. Der Gasbeutel 150, d.h. die Hauptkammer 15, ist mittels einer Fluidverbindung mit der Vorkammer 14 verbunden.

Erkennbar ist auch ein Raumluftventil 16, welches anstatt an der Halterung 15A (Bag T) für die Hauptkammer 15 befestigt zu sein abseits der Hauptkammer 15 angeordnet ist, um zu vermeiden, dass durch den Patienten während der Behandlung, jedoch insbesondere auch beim Einatmen Speichel, Blut, etc.in das Bag T in den Beutel (z. B. Spritzwasser etc.) gelangen. Insbesondere kann aus der Figur 1B erkannt werden, dass das Raumluftventil 16 an einer weiteren Halterung 15B angeordnet ist und in Richtung der Hauptkammer 15, beispielsweise auch in Richtung einer Längsachse L des Lachgasmischers 100, schließ- und öffenbar ist. Wird nun die Hauptkammer 15 durch den Patienten leergeatmet, öffnet während eines Einatmens des Patienten nach dem, insbesondere vollständigen, Entleeren der Hauptkammer 15 automatisch das Raumluftventil 16, so dass eine Fluidleitung, welche zwischen einem Nasenmaskenanschluss 17 des Lachgasmischers 1 und der Hauptkammer 15 geführt ist mit Umgebungsluft gefüllt wird. Der Patient ist somit stets ausreichend mit Sauerstoff versorgt.

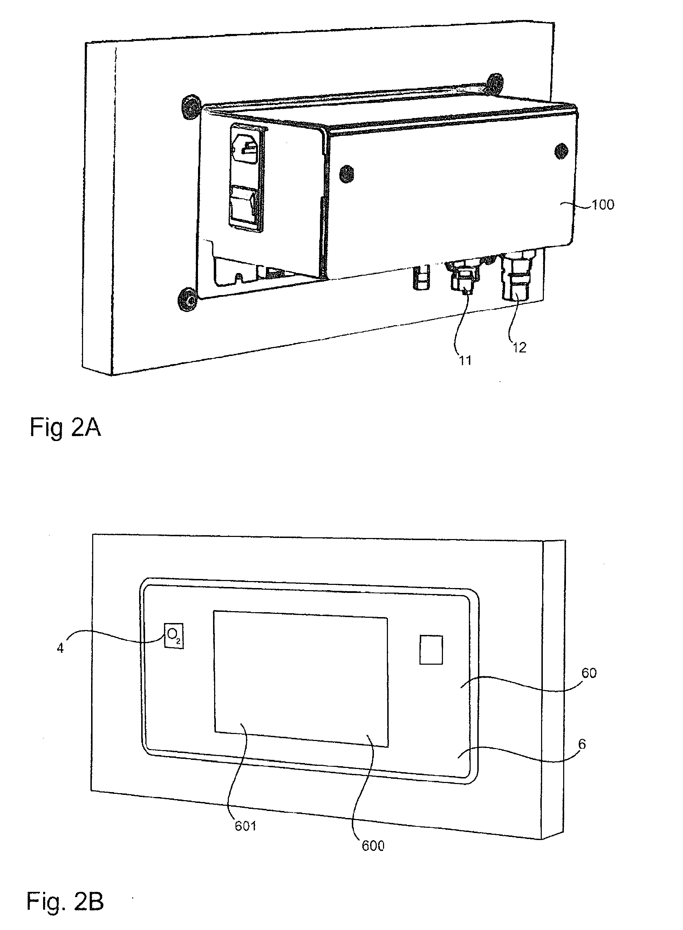

In den Figuren 2A und 2B ist in einer schematisch perspektivischen Ansicht ein Ausführungsbeispiel eines hier beschriebenen digitalen Lachgasmischers 100 gezeigt.

Wie aus der perspektivischen Rückansicht gemäß der Figur 2A erkannt werden kann, ist der beispielsweise wie in den Figuren 1A, 1B gezeigte Lachgasmischer 100 in einem Gehäuse eingebracht, wobei die verschiedenen Anschlüsse aus dem Gehäuse herausragen, um mit den entsprechenden Gasleitungen verbunden zu werden.

In der Figur 2B ist eine Vorderansicht erkennbar, in welcher eine Mischungssteuerungseinheit 6 dargestellt ist, welche eine digitale Steuerungseinheit 60 umfasst. Dabei ist die digitale Steuerungseinheit 60 derart ausgestaltet, dass diese den Konzentrationsregler 2 und/oder den Flussmengenregler 3 mittels der Vorgabe eines Benutzers über Drücken der Tasten eines Betätigungsbildschirmes 600 steuern kann. Die hier beschriebene digitale Steuerungseinheit 60 umfasst nämlich einen Betätigungsbildschirm 600, mittels dessen über eine händische Betätigung des Benutzers elektronisch, vorzugsweise nur elektronisch, der Konzentrationsregler und/oder der Flussmengenregler steuer- und/oder regelbar ist. Dies kann heißen, dass über elektronische Signale, welche von dem Betätigungsbildschirm an eine Stelleinheit gesendet werden, diese Stelleinheit dann, beispielweise mechanisch, den Konzentrationsregler und den Flussmengenregler betätigt.

Insbesondere umfasst nämlich der Betätigungsbildschirm 600 zumindest ein Einstellfeld 601, auf dem der Benutzer händisch eine Konzentrationsänderung des Sauerstoffgases 11 und/oder des Lachgases 12 in vorgebbaren Änderungsschritten einstellen kann, wobei die Konzentrationsänderung in dem vorliegenden Ausführungsbeispiel nur durch die Betätigung des Einstellfeldes 601 einstellbar ist. Erkennbar ist jedoch jedenfalls wiederum der O₂-Notfallknopf 4, welcher abgesehen von einem Ein- und Ausschaltknopf der einzige Knopf innerhalb der Montagefläche 42 des Betätigungsfeldes 600 ist, so dass auch bei dem digitalen Lachgasmischer 100 es erfindungsgemäß gewährleistet ist, dass in einem Notfall der behandelnde Arzt besonders schnell den Patienten mit Sauerstoff fluten kann.

Die Erfindung ist nicht anhand der Ausführungsbeispiele beschränkt, vielmehr umfasst die Erfindung jedes neue Merkmal sowie jede Kombination von Merkmalen, was auch insbesondere jede Kombination der Patentansprüche beinhaltet, auch wenn dieses Merkmal oder diese Kombination selbst nicht explizit in den Patentansprüchen oder den Ausführungsbeispielen angegeben ist.

### Bezugszeichenliste

- 1: Mischkammer
- 1A: Mischkammergehäuse
- 2: Konzentrationsregler
- 3: Flussmengenregler/Flow-Regler
- 4: O₂-Notfallknopf
- 6: Mischersteuerungseinheit
- 11: Sauerstoffgas
- 12: Lachgas
- 13: Lachgasgemisch
- 14: Vorkammer
- 15: Hauptkammer
- 15A: Halterung 15B weitere Halterung
- 16: Raumluftventil
- 41: Betätigungsrichtung
- 42: Montagefläche
- 60: Steuerungseinheit
- 100: Lachgasmischer
- 110: Sauerstoffgasanschluss
- 110A, 120A: Gasleitungen für die beiden zu mischenden Gase
- 110AA, 120AA: Flowmeter
- 120: Lachgasanschluss
- 150: Gasbeutel
- 600: Betätigungsbildschirm
- 601: Einstellfeld

## Patentansprüche

1. Lachgasmischer (100) zur Erzeugung eines Lachgasgemisches, umfassend:
- zumindest eine Mischkammer (1), innerhalb derer ein Sauerstoffgas (11) und ein Lachgas (12) in jeweils vorgebbaren Volumina und/oder Molanteilen miteinander zu dem Lachgasgemisch (13) vermischt werden, wobei
- die Mischkammer (1) zumindest einen Lachgasanschluss (120) und zumindest einen Sauerstoffgasanschluss (110) aufweist, so dass über entsprechend an diesen Anschlüssen montierte Gasleitungen (110A, 120A) sowohl das Sauerstoff- als auch das Lachgas in die Mischkammer (1) eingeleitet wird,
- zumindest einen Konzentrationsregler (2), welcher dazu eingerichtet und dafür vorgesehen ist, ein Mischungsverhältnis zwischen dem in die Mischkammer (1) eingeleiteten Sauerstoffgas (11) und/oder dem in die Mischkammer (1) eingeleiteten Lachgas (12) in dem Lachgasgemisch vorgebbar einzustellen,
- zumindest einen Flussmengenregler (3), mittels dessen ein Volumenstrom der beiden Gase (11, 12) jeweils separat und/oder gemeinsam eingestellt werden kann,
**dadurch gekennzeichnet, dass**
zur Notflutung der Mischkammer (1) mit dem Sauerstoffgas (11) und/oder mit Umgebungsluft an der Mischkammer (1) zumindest ein O₂-Notfallknopf (4) angebracht ist, der in einer Betätigungsrichtung (41) eindrückbar ist, wobei eine, vorzugsweise ebene, Montagefläche (42) des O₂-Notfallknopfes (4), zu welcher die Betätigungsrichtung (41) an zumindest einem Punkt in der Montagefläche (42) senkrecht verläuft, frei von weiteren Regelelementen, ist.

2. Lachgasmischer (100) zur Erzeugung eines Lachgasgemisches nach Anspruch 1,
**dadurch gekennzeichnet, dass**
die Mischkammer (1) eine Vorkammer (14) sowie eine Hauptkammer (15) umfasst, wobei nur die Vorkammer (14) die beiden Gasanschlüsse (110, 120) umfasst und die Hauptkammer (15) über zumindest eine Fluidverbindung (130) mit der Vorkammer (14) verbunden ist, wobei ein Raumluftventil (16) anstatt an einer Halterung für die Hauptkammer befestigt zu sein abseits dieser Halterung angeordnet ist.

3. Lachgasmischer (100) zur Erzeugung eines Lachgasgemisches nach Anspruch 2,
**dadurch gekennzeichnet, dass**
zumindest der O₂-Notfallknopf (4), vorzugsweise jedoch auch der Konzentrationsregler (2) und der Flussmengenregler (3), an der Vorkammer (14) angeordnet sind.

4. Lachgasmischer (100) nach zumindest einem der Ansprüche 2 bis 3,
**dadurch gekennzeichnet, dass**
die Hauptkammer (15) mit zumindest einem Gasbeutel (150) gebildet ist.

5. Lachgasmischer (100) nach zumindest einem der Ansprüche 2 bis 4,
**gekennzeichnet, durch**
zumindest eine Mischersteuerungseinheit (6), wobei die Mischersteuerungseinheit (6) zumindest eine digitale Steuerungseinheit (60) umfasst, wobei die Steuerungseinheit (60) derart ausgestaltet ist, dass diese den Konzentrationsregler (2) und/oder den Flussmengenregler (3) mittels der Vorgabe eines Benutzers steuert.

6. Lachgasmischer (100) nach dem vorhergehenden Anspruch,
**dadurch gekennzeichnet, dass**
die digitale Steuerungseinheit (60) zumindest einen Betätigungsbildschirm (600) umfasst, mittels dessen über eine händische Betätigung des Benutzers elektronisch, vorzugsweise nur elektronisch, der Konzentrationsregler (2) und/oder der Flussmengenregler (3) betätigbar sind.

7. Lachgasmischer (100) nach dem vorhergehenden Anspruch,
**dadurch gekennzeichnet, dass**
der Betätigungsbildschirm (600) zumindest ein Einstellfeld (601) umfasst, auf dem der Benutzer händisch eine Konzentrationsänderung des Sauerstoffgases (11)
und/oder des Lachgases (12) in vorgebbaren Änderungsschritten einstellen kann, wobei die Konzentrationsänderung, vorzugsweise nur, durch die Betätigung dieses Einstellfeldes (601) einstellbar ist.

8. Lachgasmischer (100) nach zumindest einem der vorhergehenden Ansprüche,
**dadurch gekennzeichnet, dass**
der Lachgasmischer neben dem O₂-Notfallknopf (4) zudem eine Lachgassperre, ein Limitierungselement der Lachgaskonzentration umfasst.

9. Lachgasmischer (100) nach zumindest einem der vorhergehenden Ansprüche,
**dadurch gekennzeichnet, dass**
der Lachgasmischer frei von einem An-und Ausschaltknopf ist.
